# EUROPEAN PATENT APPLICATION

(11) **EP 4 624 969 A1**
(43) Date of publication of application: **01.10.2025**
(21) Application number: 25165619.5
(22) Date of filing: 24.03.2025
(51) Int. Cl.: G01R 33/34

(54) **MRI HEAD COIL ADAPTOR, A KIT COMPRISING THE MRI HEAD COIL ADAPTER AND METHOD OF SECURING A PATIENTS'S HEAD RELATIVE TO AN MRI HEAD COIL.**

(30) Priority: 25.03.2024 GB 202404249
(71) Applicant: Elekta Instrument AB, 103 93 Stockholm (SE)
(72) Inventor: PLANTZ, Marianne, S-103 93 Stockholm (SE); ARN, Thomas, S-103 93 Stockholm (SE)
(74) Representative: Finlayson, Scott Henry

(57) **Abstract**

The present disclosure relates to an MRI head coil adaptor for a stereotactic frame. The MRI head coil adaptor comprising: a first part comprising an attachment portion, the attachment portion configured for attachment to a stereotactic frame or to a head fixation pin for a stereotactic frame; and a second part coupled to the first part, the second part being height-adjustable relative to the attachment portion.

## Description

### Technical Field

The present disclosure relates to an MRI head coil adaptor, to a kit comprising a head fixation pin and an MRI head coil adaptor, and to a method of using the same.

### Background

For neurosurgery and neurological radiosurgery, for example stereotactic radiosurgery, it is important before beginning treatment to obtain an accurate 3D picture of the brain, and to ensure that a mapping between the frame of reference of the 3D image and the frame of reference of the patient's brain is accurately known. Performing these preliminary steps accurately is essential to ensuring that healthy brain tissue is protected during treatment.

MRI imaging is commonly used to obtain a 3D image of the brain. During MRI imaging of the brain, the patient's head is placed inside an MRI head coil. The MRI head coil is an essential element of the MRI apparatus, acting as an antenna for receiving RF signals coming from the patient's head in order to generate the 3D image. As with any MRI imaging procedure, it is essential that the patient's head remains still relative to the MRI head coil during the imaging process.

In order to establish a known fixed frame of reference relative to the patient's brain, a stereotactic frame is worn by the patient during the imaging procedure. Furthermore, the stereotactic frame remains attached to the patient's head following the imaging procedure and for the duration of the subsequent treatment procedure. Accordingly, it is the stereotactic frame which ensures a stable and fixed frame of reference for the patient's head, such that the location of tissue to be treated can be accurately identified using the 3D image and the known mapping between the coordinate system of the 3D image and the frame of reference of the stereotactic frame. In essence, the stereotactic frame provides a reference frame relative to which the coordinate system of the 3D image can be defined.

In summary, in order to accurately and reliably perform MRI imaging for the purpose of neurosurgery and neurological radiosurgery, it is important for the stereotactic frame to have a fixed position relative to the patient's head, and for movement of the patient's head to be prevented during MRI imaging.

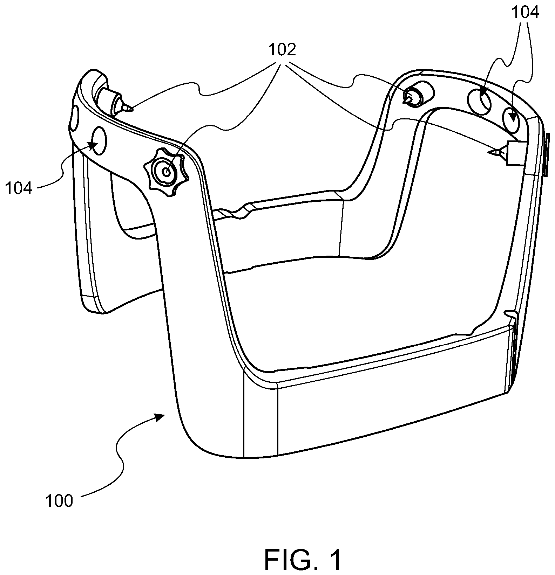

An example stereotactic frame 100 is shown in Fig. 1. During an MRI imaging procedure, the stereotactic frame 100 is first positioned around the patient's head and secured in place. The stereotactic frame 100 is secured in place by head fixation pins 102 which are inserted into apertures 104 in the stereotactic frame 100, and which each include a distal tip which presses against the patient's skull in order to secure the stereotactic frame 100 in place and prevent movement of the stereotactic frame 100 relative to the patient's head.

In the depicted example of Fig. 1, four head fixation pins 102 are used (two at the rear of the frame 100 and two at the front of the frame 100). However, in some examples, more than four pins 102, or fewer than four pins 102, may be used. Further, as shown in Fig. 1, more than four apertures 104 are present. In an exemplary example, eight apertures 104 may be present (four at the front of the frame 100, and four at the rear of the frame 100). Accordingly, the positions of the pins 102 can be selected according to the shape of the patient's head.

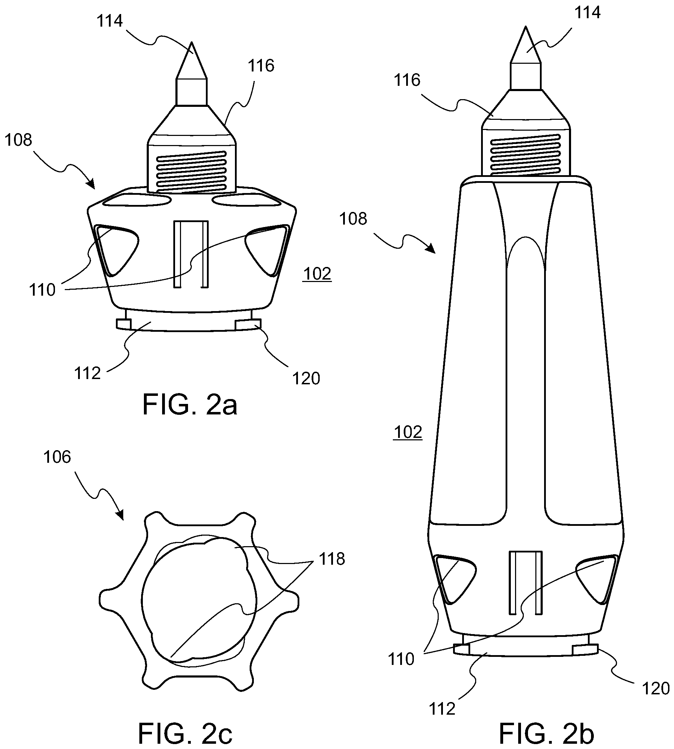

Fig. 2a shows a first example of a head fixation pin 102. Fig. 2b shows a second example of a head fixation pin 102. Fig. 2c shows an attachment ring 106 for use with a head fixation pin 102 according to Fig. 2a or 2b.

Each head fixation pin 102 includes an attachment portion 108 at a first end thereof. The attachment portion 108 includes attachment surfaces 110 for engaging an inner edge of a corresponding aperture 104 in the stereotactic frame 100. In order to initially secure an attachment pin 102 to the stereotactic frame 100, the attachment portion 108 is inserted into an aperture 104 such that the attachment surfaces 110 abut the inner surface of the stereotactic frame 100, and the attachment ring 106 is secured to the proximal end 112 of the attachment portion 108 such that the attachment ring 106 abuts the outer side of the stereotactic frame 100, thereby securing the head fixation pin 102 to the stereotactic frame 100.

As shown, the distal end of each head fixation pin 102 comprises a distal tip 114 (e.g. a pointed metallic tip 114) for engaging a patient's skull. With the head fixation pin 102 attached to the stereotactic frame 100 as described above, the distal tip 114 points inwards towards the patient's skull. The distal tip 114 is mounted to an intermediate portion 116. The intermediate portion is threaded relative to the attachment portion 108, such that the extent of projection of the distal tip 114 from the attachment portion 108 can be increased or decreased by rotating the intermediate portion 116 relative to the attachment portion 108. This enables the position of the distal tip 114 to be adjusted in order to press against the patient's skull with sufficient force to secure the stereotactic frame 100 in place (for example, with a force of at least 500N for most adult patients, or a lower force where the patient is a child or the patient's skull is otherwise weakened). Further, as shown in the figures, the head fixation pin 102 of Fig. 2b has a longer attachment portion 108 than that of the head fixation pin 102 of Fig. 2a. Accordingly, the head fixation pin 102 of Fig. 2b may be better suited for securing the stereotactic frame 100 to the head of a child.

In the present disclosure, a proximal direction is defined as a direction that is towards a stereotactic frame 100 (e.g. radially outwards and towards a surface of a stereotactic frame, or radially inwards and towards a surface of a stereotactic frame 100). Further, a distal direction is defined as a direction that is away from a stereotactic frame (e.g. either radially inwards and away from a surface of a stereotactic frame, or radially outwards and away from a surface of a stereotactic frame).

As shown in Fig. 2c, the attachment ring 106 comprises a female bayonet attachment 118 configured to engage a corresponding male bayonet attachment 120 at the proximal end of the attachment portion 108. Accordingly, the attachment ring 106 is attached to the attachment portion 108 by rotating the attachment ring 106 relative to the attachment portion 108.

Once the stereotactic frame 100 has been secured to the patient's head using head fixation pins 102 as described above, a fiducial frame may be attached to it. The fiducial frame may include fiducial markers for calibrating the coordinate system of the 3D MRI image relative to the frame of reference of the stereotactic frame 100. In other examples, fiducial markers may be present on the stereotactic frame 100 itself, thus dispensing with the need for a separate fiducial frame. Where the fiducial frame is for use in MRI imaging, the fiducial markers may comprise a material which is relatively opaque to MRI imaging, for example a channel filled with water, optionally further including copper sulphate (CuSO4).

In order to perform MRI imaging, the patient's head (with the stereotactic frame 100 attached thereto) must be positioned within an MRI head coil for imaging. In order to prevent movement of the patient's head relative to the MRI head coil (which would negatively affect the quality and accuracy of the image), padding may be wedged between the stereotactic frame and the MRI head coil. A problem with this approach, however, is that the use of such padding is cumbersome, may cause discomfort for the patient, and may not completely secure the patient's head against movement. Additionally, such padding may also risk dislocating the stereotactic frame as it is installed, e.g. by pushing the stereotactic frame relative to the patient's head thereby causing dislocation of the frame. Furthermore, such padding can deform or dislodge the fiducial frame relative to the stereotactic frame.

In another example, a stereotactic frame which is specific to the MRI head coil may be used, and may attach directly to the MRI head coil to prevent movement. However, because there are a range of different MRI head coils provided by different manufacturers and for different imaging requirements, lack of compatibility is a challenge for such stereotactic frames. It is further undesirable for a hospital to have to provide a different specific stereotactic frame for use with each different MRI head coil.

The present invention builds on the state of the art as described above, and has in particular been developed to address at least some of the problems noted above.

### Summary

According to a first aspect of the present disclosure, there is provided an MRI head coil adaptor for a stereotactic frame, the MRI head coil adaptor comprising: a first part comprising an attachment portion, the attachment portion configured for attachment to a stereotactic frame or to a head fixation pin for a stereotactic frame; and a second part coupled to the first part, the second part being height-adjustable relative to the attachment portion.

By providing an adaptor which couples directly to the stereotactic frame (or to the head fixation pin), improved stability of the frame relative to the MRI head coil can be achieved. Further, by providing an adaptor in which the height of the second portion relative to the attachment portion is adjustable, the adaptor enables a single stereotactic frame to be used with a variety of different MRI head coils. That is to say, compatibility may be improved.

The height-adjustability may be achieved by a threaded arrangement. For example, the second part may be threaded onto the first part, such that the height of the second part relative to the attachment portion is adjustable by rotating the second part relative to the first part.

The attachment portion may be configured for attachment to a head fixation pin for a stereotactic frame. Alternatively, the attachment portion may be configured for attachment to a stereotactic frame at a location on the stereotactic frame configured to receive a head fixation pin. For example, the present disclosure may provide a system comprising a stereotactic frame and a head coil adaptor according to the first aspect, wherein the attachment portion of the head coil adaptor is configured for attachment to a corresponding attachment surface on the stereotactic frame, wherein the corresponding attachment surface on the stereotactic frame is further configured for attachment to a head fixation pin.

The attachment portion may comprise a bayonet attachment, for example a female bayonet attachment, or a male bayonet attachment. Advantageously, such a bayonet attachment may be configured to couple to a cooperating bayonet attachment on the proximal end of a head fixation pin. In other examples, the bayonet attachment may be configured to couple to a cooperating bayonet attachment on the stereotactic frame.

Each of the first part and the second part may be a non-conductive and non-metallic material, such as a non-metallic material. For example, at least one of the first part and the second part may be formed of a polymer material. Accordingly, the adaptor may be substantially 'transparent' to the MRI imaging.

The first part may comprise a substantially rigid polymer, for example polyoxymethylene ("POM"), polyamide ("PA"), or a glass fiber composite comprising a POM or PA matrix. Accordingly, the first part may provide a stable and rigid attachment point for the second part, which may in turn help to improve stability of the patient's head relative to an MRI head coil.

At least the threaded portion of the second part may comprise a substantially rigid polymer, for example polyoxymethylene ("POM"), polyamide ("PA"), or a glass fiber composite comprising a POM or PA matrix . At least an upper surface of the second portion may comprise a deformable polymer material, for example rubber. This may help to provide improved friction and stability between the upper surface and an MRI head coil against which the upper surface abuts in use.

In a second aspect there is provided a kit comprising: a head fixation pin; and an MRI head coil adaptor according to the first aspect; wherein the head fixation pin is configured for attachment to a stereotactic frame; and wherein the MRI head coil adaptor is configured for attachment to the head fixation pin.

By providing attachment of the MRI head coil adaptor directly to the head fixation pin, there is accordingly a direct engagement between the MRI head coil adaptor and the patient's skull, thereby ensuring maximum stability between the patient's brain and the MRI head coil during imaging.

The head fixation pin may comprise one of a male bayonet attachment and a female bayonet attachment. The attachment portion of the MRI head coil adaptor may comprise the other of a male bayonet attachment and a female bayonet attachment.

The head fixation pin may comprise an attachment portion at a first end thereof. The head fixation pin may comprise a distal tip for engaging a patient's head at a second end thereof. The attachment portion may be configured for attachment to a stereotactic frame and to the MRI head coil attachment.

The attachment portion of the head fixation pin may comprise an attachment surface for engaging a stereotactic frame. The attachment surface may be for insertion into a corresponding aperture of the stereotactic frame.

The attachment portion of the head fixation pin may comprise the one of the male bayonet attachment and the female bayonet attachment.

In a third aspect there is provided a method of securing a patient's head relative to an MRI head coil, the method comprising:
arranging a stereotactic frame around the patient's head;
securing a head fixation pin to the stereotactic frame, such that the head fixation pin contacts the patient's head and such that the stereotactic frame is secured to the patient's head;
attaching an MRI head coil adaptor according to the first aspect to the head fixation pin, or to the stereotactic frame at a location adjacent the head fixation pin;
placing the patient's head within an MRI head coil; and
adjusting a height of the second part of the MRI head coil adaptor such that the second part presses against the MRI head coil.

In a fourth aspect there is provided a method of securing a patient's head relative to an MRI head coil, the method comprising:
arranging a stereotactic frame around the patient's head;
securing a head fixation pin of the kit according to the second aspect to the stereotactic frame, such that the head fixation pin contacts the patient's head and such that the stereotactic frame is secured to the patient's head;
attaching a MRI head coil adaptor of the kit according to the second aspect to the head fixation pin;
placing the patient's head within an MRI head coil; and
adjusting a height of the second part of the MRI head coil adaptor such that the second part presses against the MRI head coil.

In a fifth aspect there is provided a kit comprising an MRI head coil adaptor according to the first aspect, and a further second part for coupling to the first part, wherein the further second part is taller than the second part, and wherein the second part coupled to the first part is replaceable by the further second part. The further second part may, apart from its increased height, be in all other respects identical to the second part. For example, the further second part may be height-adjustable relative to the attachment portion of the first part, for example by rotating the second part relative to the first part. The further second part may also be non-metalling, for example formed of a polymer as described above. The further second part may also comprise a deformable polymer material at an upper surface thereof. Replacing the second part with the further second part may comprise unthreading the second part from the first part, and then threading the further second part onto the first part.

### Brief Description of the Drawings

Examples of the present disclosure will now be described, with reference to the accompanying drawings, in which:
Fig. 1 shows a known stereotactic frame;
Fig. 2a shows a first type of known head fixation pin for use with the stereotactic frame of Fig. 1;
Fig. 2b shows a second type of known head fixation pin for use with the stereotactic frame of Fig. 1;
Fig. 2c shows an attachment ring for use with a head fixation pin according to Fig. 2a or 2b;
Fig. 3 shows an MRI head coil adaptor according to the present disclosure;
Fig. 4a shows a perspective view of a first part of the MRI head coil adaptor of Fig. 3;
Fig. 4b shows an end-view of the first part of Fig. 4a;
Fig. 4c shows a perspective view of a second part of the MRI head coil adaptor of Fig. 3;
Fig. 4d shows a perspective view of an optional further second part of the MRI head coil adaptor of Fig. 3;
Fig. 5 shows the MRI head coil adaptor of Fig. 3 coupled to the known stereotactic frame of Fig. 1;
Fig. 6a shows a first type of MRI head coil having the stereotactic frame of Fig. 1 secured therein using the MRI head coil adaptor of Fig. 3;
Fig. 6b shows a second type of MRI head coil having the stereotactic frame of Fig. 1 secured therein using the MRI head coil adaptor of Fig. 3; and
Fig. 7 shows a method of securing a patient's head relative to an MRI head coil using the MRI head coil adaptor of Fig. 3.

Like reference numerals are used for like components throughout the drawings.

### Detailed Description

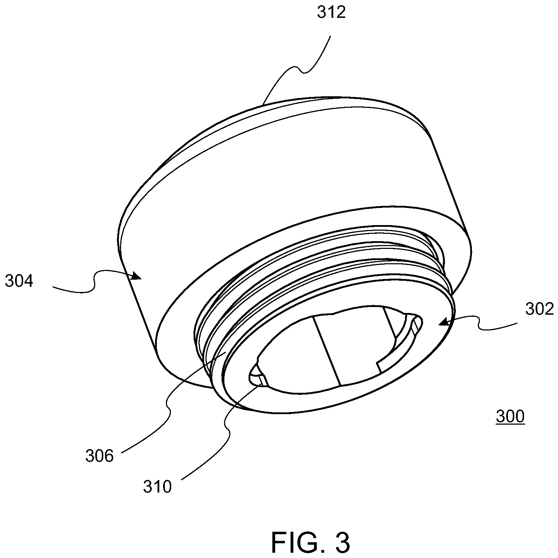

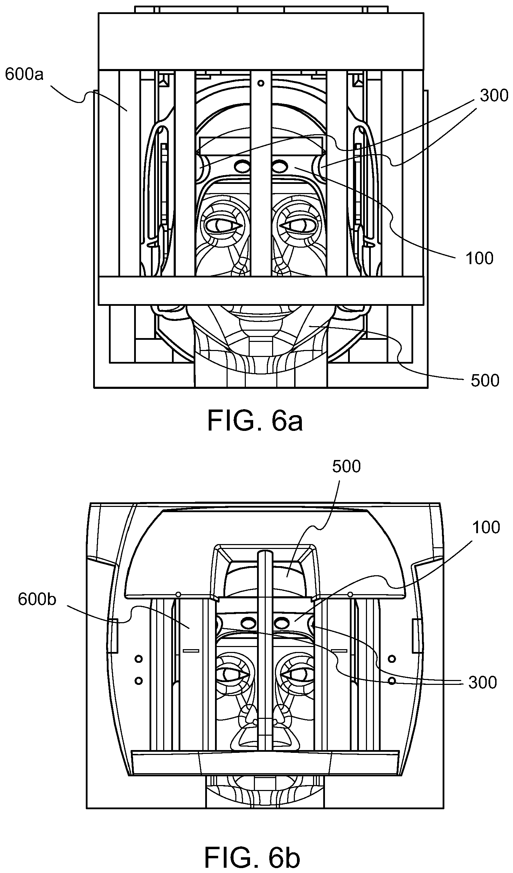

Fig. 3 shows an MRI head coil adaptor 300 according to the present disclosure. The MRI head coil adaptor 300 is configured for attachment to a stereotactic frame, for example a stereotactic frame 100 for use in neurological radiosurgery as shown in Fig. 1. In particular, the MRI head coil adaptor 300 attaches to a stereotactic frame and is adjustable in order to provide adaptable engagement with a variety of different MRI head coils, for example with an MRI head coil 600a of a first type or with an MRI head coil 600b of a second type. MRI head coils 600a, 60b will be described further in relation to Figs. 6a and 6b.

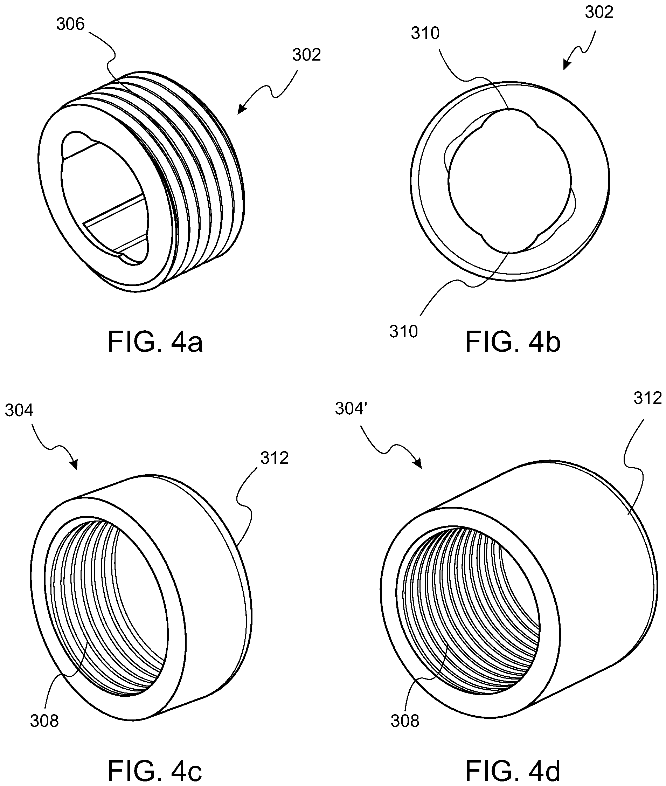

Returning to Fig. 3, the MRI head coil adaptor 300 according to the present disclosure comprises a first part 302, and a second part 304. Figs. 4a and 4b show the first part 302 on its own (i.e. with the second part 304 removed therefrom). Fig. 4c shows the second part 304 on its own (i.e. with the first part 302 removed therefrom). The first part 302 comprises a first thread 306 on an outer surface thereof, and the second part 304 comprises a cooperating second thread 308 on an inner surface thereof. Accordingly, by rotating the second part 304 relative to the first part 302, the height of the second part 304 relative to the first part 302 can be increased and decreased. For example, rotating the second part 304 in a first direction relative to the second part 302 may decrease the height of the second part 304 relative to the first part 302, and rotating the second part 304 in a second direction relative to the first part 302 may increase the height of the second part 304 relative to the first part 302.

The first part 302 as shown in Figs. 4a (perspective view) and 4b (end-view) will now be described in more detail. The first part 302 is formed of a rigid polymer, such as POM, PA, or a glass fiber composite thereof. The rigidity of these materials makes them well-suited for securing the stereotactic frame 100 relative to the MRI head coil. Furthermore, they are chemically inert, and are therefore well-suited to medical applications. Further, polymers interfere minimally with MRI imaging. As the reader will understand, however, any suitable substantially rigid, non-conductive, and non-magnetic material could be used.

As shown in Figs. 4a and 4b, the first part 302 comprises a substantially cylindrical tube, which is hollow and is open at each end. The first part 302 comprises the first thread 306 on the outer surface thereof. Furthermore, an inner surface of the first part 302 comprises a female bayonet attachment 310. The female bayonet attachment is located at a proximal end of the first part 302 (and by extension at a proximal end of the MRI head coil adaptor 300). The female bayonet attachment 310 is configured to cooperate with the male bayonet attachment 120 located on the attachment portion 108 of a head fixation pin 102. Accordingly, the first part 302 is attachable securely to a head fixation pin 102 using the cooperating bayonet attachments. In the case where the head fixation pin 102 is mounted to a stereotactic frame 100, the first part 302 is accordingly attached securely to the stereotactic frame 100. The first part 302 may therefore be a base part 302 of the MRI head coil adaptor 300.

The second part 304 as shown in Fig. 4c will now be described in more detail. The second part 304 is formed of a rigid polymer, such as POM, PA, or a glass fiber composite thereof. Optionally, however, a top (distal) surface 312 of the second part 304 may comprise a deformable polymer material, such as rubber.

As shown in Fig. 4c, the second part 304 comprises a substantially cylindrical tube, which is open at a proximal end thereof in order to receive the first part 302 therein; and a distal end which is closed. The second part 304 comprises the second thread 308 on the inner surface thereof, for engaging the thread 306 of the first part 302. The distal end surface 312 is convexly curved.

Fig. 4d shows an optional further second part 304', which may be provided with the first part 302 and the second part 304 in a kit. As shown, the further second part 304' has the same structure as the second part 304, including the internal thread 308 and the optionally deformable curved top surface 312. However, the further second part 304' is taller than the second part 304. Accordingly, depending on the spacing distance to be bridged between the stereotactic frame 100 and MRI head coil, an appropriate one of the second part 304 and the further second part 304' can be selected for attachment to the first part 302.

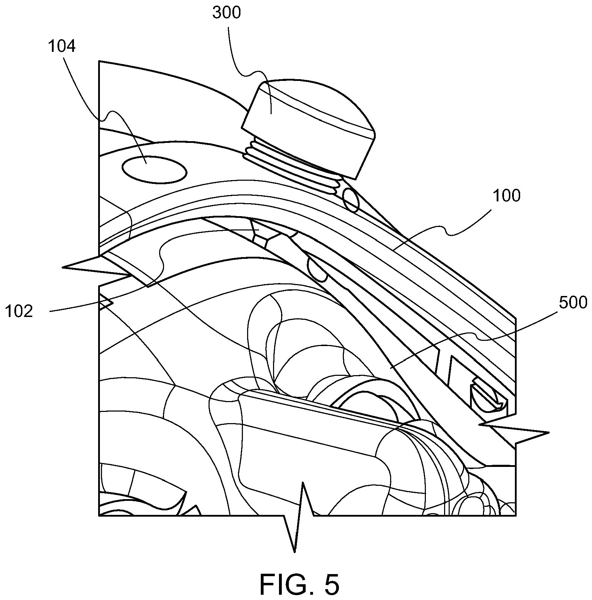

Fig. 5 shows the MRI head coil adaptor 300 of Fig. 3 attached to a stereotactic frame 100. Shown in Fig. 5 is the stereotactic frame 100, a head fixation pin 102, and the MRI head coil adaptor 300. As can be seen, the head fixation pin 102 extends radially inwards from the stereotactic frame 100, for contacting the patient's head 500. The MRI head coil adaptor 300 extends radially outwards from the stereotactic frame 100, for contacting an MRI head coil (not shown in Fig. 5).

Fig. 6a shows an MRI head coil 600a of a first type with a patient's head 500 received therein. As shown, two MRI head coil adaptors 300 are used. Each head coil adaptor 300 presses against an inner surface of the MRI head coil 600a so as to secure the patient's head in place.

Fig. 6b shows an MRI head coil 600b of a second type with a patient's head 500 received therein. As shown, two MRI head coil adaptors 300 are used. Each head coil adaptor 300 presses against an inner surface of the MRI head coil 600b so as to secure the patient's head in place.

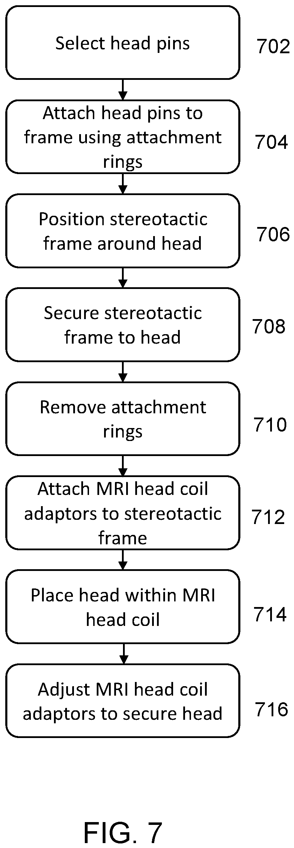

Fig. 7 illustrates a method of securing a patient's head within an MRI head coil (e.g. an MRI head coil 600a of a first type, or an MRI head coil 600b of a second type).

At step 702, a patient's head is measured. Based on the measurement, head fixation pins 102 suitable to the patient's head size are selected. For example, if the patient's head is below a predefined diameter, head fixation pins 102 as shown in Fig. 2b are selected. If the patient's head is above the predefined diameter, head fixation pins 102 as shown in Fig. 2a are selected.

At step 704, the head fixation pins 102 are secured to a stereotactic frame 100. In particular, each head fixation pin 102 is secured to the stereotactic frame 100 by passing the attachment portion 108 through a respective aperture 104 and then securing the attachment ring 106 to the proximal end 112 of the attachment portion 108 in order to thereby secure the head fixation pin 102 to the stereotactic frame 100. For example, four head pins 102 may be secured to the stereotactic frame 100 using respective attachment rings 106.

At step 706, the stereotactic frame 100 (with the head fixation pins 102 attached thereto) is positioned around the patient's head. Optionally, this step may be preceded by a step of applying anaesthetic to the areas on the patient's head which will be engaged by the head fixation pins 102.

At step 708, the intermediate portions 116 of each of the head fixation pins 102 are rotated until the distal pointed tips 114 press against the patient's skull with sufficient force (e.g. 500N) to secure the stereotactic frame in place, thereby securing the stereotactic frame in place.

At step 710, the attachment rings 106 are removed from the proximal ends 112 of the attachment portions 108, thereby freeing up the male bayonet attachment portions 120.

At step 712, an MRI head coil adaptor 300 is attached to the proximal end of each attachment portion 108, using the bayonet attachments 120, 310.

At step 714, the patient's head (with the stereotactic frame secured thereto) is placed within the MRI head coil. Where a fiducial frame is used for the MRI imaging, the fiducial box is secured to the stereotactic frame prior to insertion of the patient" head into the MRI head coil. For example, the fiducial frame may be secured to the stereotactic frame at any suitable point between steps 708 and 714.

At step 716, the second portion 304 of each MRI head coil adaptor 300 is rotated until each MRI head coil adaptor 304 presses against the MRI head coil with sufficient force to secure the patient's head in place.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. An MRI head coil adaptor for a stereotactic frame, the MRI head coil adaptor comprising:
a first part comprising an attachment portion, the attachment portion configured for attachment to a stereotactic frame or to a head fixation pin for a stereotactic frame; and
a second part coupled to the first part, the second part being height-adjustable relative to the attachment portion.

2. The MRI head coil adaptor of claim 1, wherein the second part is threaded onto the first part, such that the height of the second part relative to the attachment portion is adjustable by rotating the second part relative to the first part.

3. The MRI head coil adaptor of claim 1 or claim 2, wherein the attachment portion comprises a bayonet attachment.

4. The MRI head coil adaptor of any preceding claim, wherein each of the first part and the second part is non-metallic.

5. The MRI head coil adaptor of any preceding claim, wherein at least one of the first part and the second part is formed of a polymer material.

6. The MRI head coil adaptor of any preceding claim, wherein at least an upper surface of the second portion comprises a deformable polymer material.

7. The MRI head coil adaptor of any preceding claim, wherein the first part comprises a substantially rigid polymer.

8. The MRI head coil adaptor of any preceding claim, wherein at least the threaded portion of the second part comprises a substantially rigid polymer.

9. The MRI head coil adaptor of any preceding claim, wherein the attachment portion is configured for attachment to a head fixation pin for a stereotactic frame, or wherein the attachment portion is configured for attachment to a stereotactic frame at a location on the stereotactic frame configured to receive a head fixation pin.

10. A kit comprising:
a head fixation pin; and
an MRI head coil adaptor according to any preceding claim;
wherein the head fixation pin is configured for attachment to a stereotactic frame; and
wherein the MRI head coil adaptor is configured for attachment to the head fixation pin.

11. The kit of claim 10, wherein the head fixation pin comprises one of a male bayonet attachment and a female bayonet attachment, and wherein the attachment portion of the MRI head coil adaptor comprises the other of a male bayonet attachment and a female bayonet attachment.

12. The kit of claim 10 or claim 11, wherein the head fixation pin comprises an attachment portion at a first end thereof, and further comprises a distal tip for engaging a patient's head at a second end thereof, wherein the attachment portion is configured for attachment to a stereotactic frame and to the MRI head coil attachment.

13. The kit of claim 12, wherein the attachment portion of the head fixation pin comprises an attachment surface for engaging a stereotactic frame, for example wherein the attachment surface is for insertion into a corresponding aperture of the stereotactic frame.

14. The kit of claim 12 or claim 13 when dependent upon claim 11, wherein the attachment portion of the head fixation pin comprises the one of the male bayonet attachment and the female bayonet attachment.

15. A method of securing a patient's head relative to an MRI head coil, the method comprising:
arranging a stereotactic frame around the patient's head;
securing a head fixation pin to the stereotactic frame, such that the head fixation pin contacts the patient's head and such that the stereotactic frame is secured to the patient's head;
attaching an MRI head coil adaptor according to any of claims 1 to 9 to the head fixation pin, or to the stereotactic frame at a location adjacent the head fixation pin;
placing the patient's head within an MRI head coil; and
adjusting a height of the second part of the MRI head coil adaptor such that the second part presses against the MRI head coil.
